# EUROPÄISCHE PATENTSCHRIFT

(11) **EP 0 938 330 B1**
(45) Veröffentlichungstag und Bekanntmachung des Hinweises auf die Patenterteilung: **06.02.2002**
(21) Anmeldenummer: 97947017.6
(22) Anmeldetag: 23.10.1997
(51) Int. Cl.: A61K 38/09

(54) **VERWENDUNG VON LH-RH ANTAGONISTEN ALS DIAGNOSTISCHES MITTEL**
USE OF LH-RH ANTAGONISTS AS DIAGNOSTIC AGENT
UTILISATION D'ANTAGONISTES DE LA L.H.-R.H. COMME AGENT DIAGNOSTIQUE

(30) Priorität: 30.10.1996 DE 19644994
(43) Veröffentlichungstag der Anmeldung: 01.09.1999
(73) Patentinhaber: Zentaris AG, 60314 Frankfurt/Main (DE)
(72) Erfinder: ENGEL, Jürgen, D-63755 Alzenau (DE); DIEDRICH, Klaus, D-23627 Gross-Sarau (DE); FELBERBAUM, Ricardo, D-23566 Lübeck (DE)
(86) Internationale Anmeldenummer: DE9702456
(87) Internationale Veröffentlichungsnummer: WO9818482

(56) Entgegenhaltungen:
- EP-A- 0 299 402
- EP-A- 0 657 170
- PH. MERVIEL ET AL.: "L'hystéroscopie opératoire en 1995. Instrumentation, technique, indications et résultats." CONTRACEPT. FERTIL. SEXUAL., Bd. 23, Nr. 9, 1995, Seiten 516-523, XP002057852 in der Anmeldung erwähnt
- L. BOUBLI ET AL.: "Préparation de l'hystéroscopie opératoire par agonistes du GnRH." CONTRACEPT. FERTIL. SEXUAL., Bd. 20, Nr. 5, 1992, Seiten 567-570, XP002057853

## Beschreibung

Die Erfindung betrifft ein diagnostisches Mittel insbesondere zur Anwendung bei der Vorbereitung der Hysteroskopie. Unter Hysteroskopie ist die endoskopische Inspektion der Gebärmutterhöhle mit einem Spezialendoskop (Hysteroskop) zu verstehen.
Die Hysteroskopie ist eine essentielle diagnostische Methode zur Beurteilung von diversen Gebärmuttererkrankungen zum Beispiel der Myome bzw. der endometrialen Hyperplasie .

Aus Gynaecol.-Endoscopy 4:4 259-264 1995 ist bekannt, GnRH -Analoga, hier Triptorelin, vor der laparskopischen Myomektomie einzusetzen.
Ferner wurde mit dem LH-RH Agonisten Tryptorelin eine Behandlung von endometrialer Hyperplasie unter hysteroskopischer und diagnostischer Curretage durchgeführt ( Contraception-Fertilite-Sexualite 23, 9, 516 - 523, (1995).
Aus J.Gynaecol.Surg, 11:2, 65 - 70, (1995) ist es bekannt, den Effekt der endometrialen Verdünnung durch Gn - RH Agonisten (Zoladex) zu bewerten im Hinblick auf die Ergebnisse der hysteroskopischen, endometrialen elektrochirurgischen Resektion. Die Präparation des Endometriums vor der hysteroskopischen Chirurgie wird empfohlen.
Aus TW. Gynaekol. 6:6,382-390 (1993) sind Ergebnisse bei der Infertilitätsbehandlung mit GnRH Analoga bekannt, wonach das Volumen der Leiomyome vor der laparaskopischen Myomektomie oder der hysteroskopischen Resektion erfolgt.

Die Verwendung von LHRH - Antagonisten, insbesondere von Cetrorelix ist bisher im Zusammenhang mit der hysteroskopischen Diagnostik oder Operationsvorbereitung nicht bekannt.

Aufgabe der Erfindung ist daher die Bereitstellung eines Mittels zur Verbesserung der Ergebnisse der Hysteroskopie im Zusammenhang mit der Diagnostik von Zuständen des Uterus insbesondere bei Endometriumhyperplasie, Leiomyomen oder bösartigen Erkrankungen. Die Erfindung ermöglicht zusätzlich eine nichtinvasive Therapie bzw.eine Operation ohne lange Vorbereitungszeit. Damit kann simultan eine Behandlung der o.g. Erkrankungen verbunden werden.

Die Aufgabe wird dadurch gelöst, daß LH-RH Antagonisten, insbesondere der beispielsweise aus EP 0 299 402 bekannte LH-RH Antagonist Cetrorelix in Vorbereitung der Hysteroskopie zur Herstellung eines diagnostischen Mittels bei den o.g. Anwendungsfällen eingesetzt wird.

Durch die unmittelbar einsetzende Wirkung von Cetrorelix ist dessen Anwendung insbesondere auch im Zusammenhang mit der Diagnose und Therapie beispielsweise bei Myomblutungen angezeigt.

Die Diagnostik kann vorteilhaft unmittelbar in die Therapie mit therapeutischer Dosen übergehen.
Die Behandlung beispielsweise der Leiomyome kann nach folgendem Schema erfolgen:

### Beispiel

In einer Ausführungsform der Erfindung wird **0,1 bis 2 mg/kg** Körpergewicht als single Dosis oder **0,01** bis 0,5 mg/kg Körpergewicht Cetrorelix beispielsweise verteilt über 1 bis 14 Tage injiziert.
Die Folge ist ein Rückgang der Schichtdicke des Endometriums und die dadurch bedingte hysteroskopisch effektivere Beurteilungsmöglichkeit und Therapie krankhafter Zustände. Durch die rasch einsetzende Wirkung von Cetrorelix ist diese Gabe insbesondere auch bei der diagnostischen Vorbereitung von solchen Operationen angezeigt, die keinen Aufschub dulden, wie beispielsweise Mammacarcinom.

Die nachfolgende Tabelle 1 zeigt den Rückgang bzw. die Stagnation des Endometrium - Wachstums verfolgt bei einer Patientin mit den Initialen 112 KB und wird im folgenden erläutert:

### Cetrorelix Pamoat: vorläufige Ergebnisse der klinischen Versuche:

Behandlung einer Patientin mit uteriner Fibrosis, die operiert wurde.
Injektion von 60 mg Cetrorelix am Zyklustag 2 und 28.
Das Endometrium wurde durch Ultraschall gemessen und zeigte kein Wachstum während der Cetrorelixbehandlung. Die hysteroskopische und laparoskopische Myom Resektion wurde einfach durchgeführt ohne einen signifikanten Blutverlust nur 2 Monate nach Beginn der Therapie mit Cetrorelix.
Für diagnostische Untersuchungen oder Ablation des Endometriums ist eine kurze Periode von 7 bis 14 Tagen mit Cetrorelix -Behandlung ausreichend wegen der sofort einsetzenden Abnahme der Schichtdicke des Endmetriums nach der Injektion von Cetrorelix.

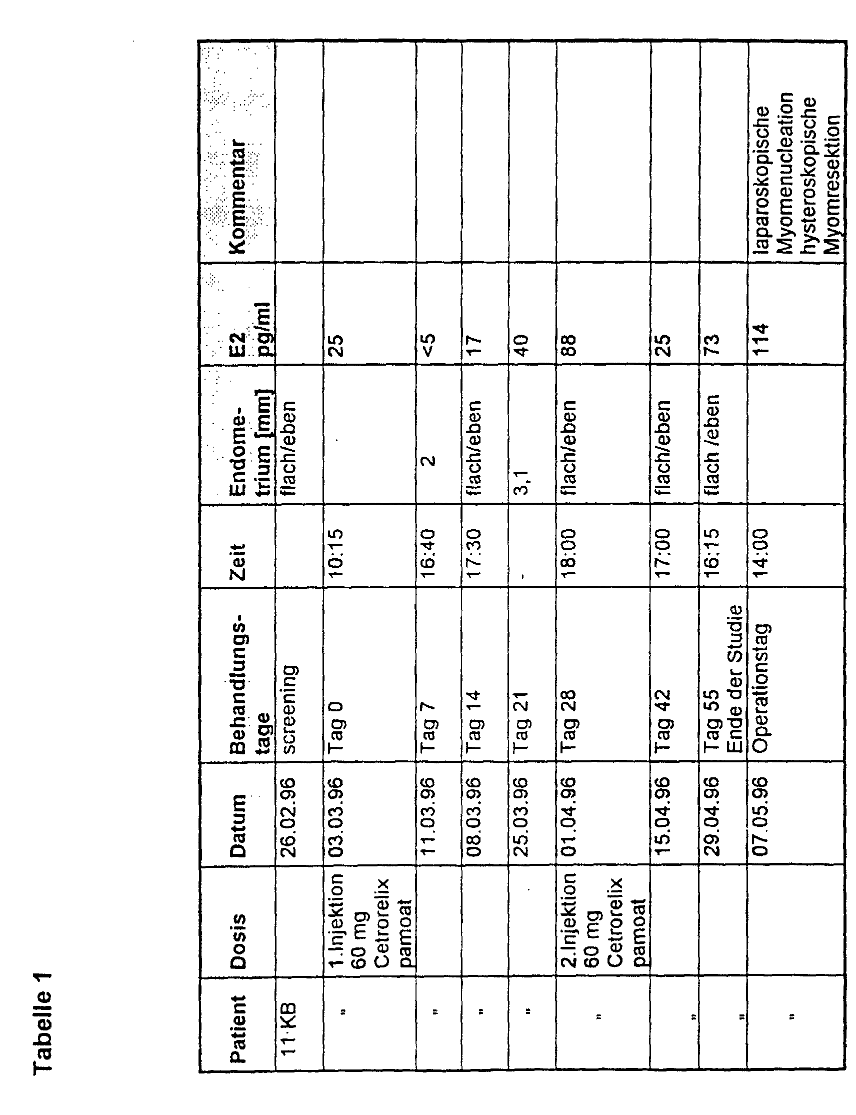

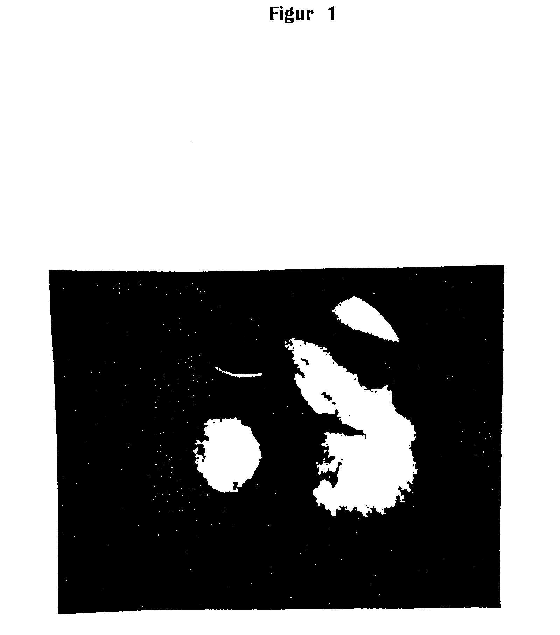

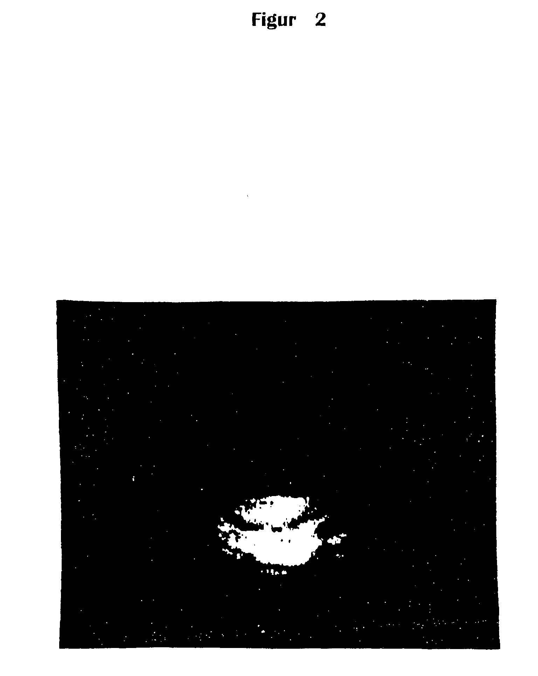

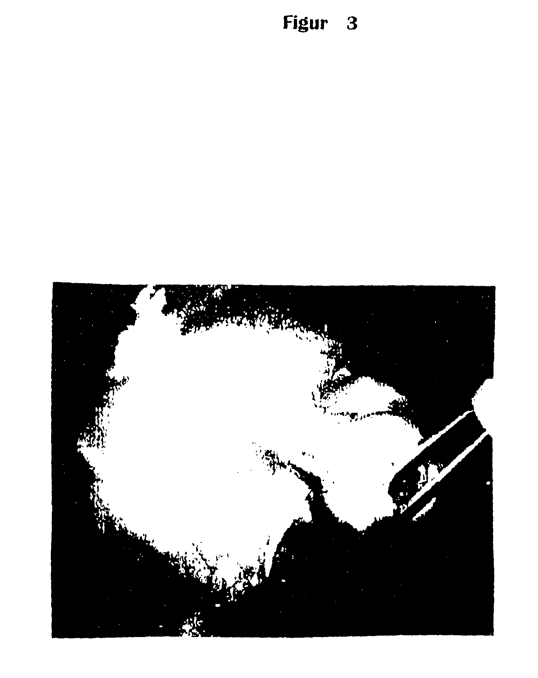

### Erläuterung der anliegenden Figuren 1 bis 3:

Figur 1 zeigt ein Operationsbild vor Beginn der hysteroskopischen Myomabtragung
Figur 2 zeigt ein Operationsbild nach hysteroskopischer Myomabtragung. Links oben ostium pubae internum
Figur 3 zeigt die Enucleation des subserösen Fundusmyoms

Die Vorteile der Erfindung bestehen vor allem darin :
→ verbessertes diagnostisches Prinzip basierend auf einer schnellen Hormonsuppression und damit eine schnelle Reduktion bzw. ein Sistieren des Endometriumvolumens und damit Verbesserung der hysteroskopischen Diagnostik sowie verkürzte Behandlungsdauer im Vergleich zu bekannten Methoden
→ sehr gute Verträglichkeit wegen fehlender initialer Stimulation (Flare up).

### Vorteile der medizinischen Vorbehandlung mit Cetrorelix sind insbesondere :

→ Behandlungsbeginn ist möglich unabhängig von Stand des Menstrualzyklus
→ Kurze Behandlungsdauer von 7 bis 10 Tagen, daher keine Nebenwirkungen und keine oder nur geringfügige Hormonmangelsymtome
→ gutes Timing der hysteroskopischen Inspektion

### Vergleich zu bestehenden Therapien

→ LHRH-Agonisten: inital flare-up
   a) lange Behandlungsdauer (4 Wochen)
   b) größere Häufigkeit von Hormonmangelerscheinungen
   c) Zunahme der Blutungen während der ersten Tage

### → Gestagene:

### unregelmäßge Blutungen

### → Danazol:

a) hohe Dosen sind erforderlich
b) lange Behandlungsdauer (4-6 Wochen)
c) Nebenwirkungen: Akne, Hirsutismus, Gewichtszunahme

## Patentansprüche

1. Verwendung von LH-RH Antagonisten zur Herstellung von diagnostischen Mitteln zur Verbesserung der Effektivität der Hysteroskopie.

2. Verwendung nach Anspruch 1, **dadurch gekennzeichnet, daß** als LH-RH Antagonist Cetrorelix eingesetzt wird.

3. Verwendung nach Anspruch 1 und 2 dadurch gekennnezeichnet, daß der LH-RH Antagonist, insbesondere Cetrorelix vor der Hysteroskopie und / oder zur Operationsvorbereitung in einer Einmalgabe zwischen 0,1 bis 2 mg/kg Körpergewicht verabreich bar ist.

4. Verwendung nach Anspruch 1 und 2 **dadurch gekennzeichnet, daß** der LH-RH Antagonist, insbesondere Cetrorelix vor der Hysteroskopie und / oder zur Operationsvorbereitung in einer Mehrfachgabe zwischen 0,01 bis 0,5 mg/kg körpergewicht, vorzugsweise verteilt über 1-14 Tage verabreich bar ist.

5. Verwendung von LH-RH Antagonisten, insbesondere von Cetrorelix zur Herstellung von diagnostischen Mitteln zur Verbesserung der Effektivität der Hysteroskopie in Kombination mit anschließender Abtragung von Myomen oder Behandlung einer Endometriumhyperplasie.

## Claims

1. Use of LH-RH antagonists for producing diagnostic compositions for improving the effectiveness of hysteroscopy.

2. Use according to Claim 1, **characterized in that** the LH-RH antagonist used is cetrorelix.

3. Use according to Claims 1 and 2, **characterized in that**, prior to hysteroscopy and/or for preparation for surgery, the LH-RH antagonist, in particular cetrorelix, can be administered in a single dose of between 0.1 and 2 mg/kg of body weight.

4. Use according to Claims 1 and 2, **characterized in that**, prior to hysteroscopy and/or for preparation for surgery, the LH-RH antagonist, in particular cetrorelix, can be administered in a multiple dose of between 0.01 and 0.5 mg/kg of body weight, preferably spread over 1-14 days.

5. Use of LH-RH antagonists, in particular cetrorelix, for producing diagnostic compositions for improving the effectiveness of hysteroscopy in combination with subsequent ablation of myomas, or treatment of an endometrial hyperplasia.

## Revendications

1. Utilisation d'antagonistes de LH/RH en vue de la préparation d'un agent diagnostique pour l'amélioration de l'efficacité de l'hystéroscopie.

2. Utilisation selon la revendication 1,
**caractérisée en ce que**
comme antagoniste de LH/RH on met en oeuvre le Cetrorelix.

3. Utilisation selon l'une des revendications 1 ou 2,
**caractérisée en ce que**
l'antagoniste de LH/RH, en particulier le Cetrorelix, peut être administré avant l'hystéroscopie et/ou en vue de la préparation de l'opération, en une administration en une fois, comprise entre 0,1 et 2 mg/Kg de poids corporel.

4. Utilisation selon l'une des revendications 1 ou 2,
**caractérisée en ce que**
l'antagoniste de LH/RH, en particulier le Cetrorelix, peut étre administré avant l'hystéroscopie et/ou en vue de la préparation de l'opération, en une administration multiple comprise entre 0,01 et 0,5 mg/kg de poids corporel, de préférence réparti sur 1 à 14 jours.

5. Utilisation d'antagoniste de LH/RH, en particulier le Cetrorelix, en vue de la préparation d'agents de diagnostic pour l'amélioration de l'efficacité de l'hystéroscopie en combinaison avec l'ablation corrélative de myomes ou avec le traitement d'une hyperplasie de l'endomètre.
